## Europäisches Patentamt
## European Patent Office
## Office européen des brevets

(19)

(11) Numéro de publication: **0152441**
**B1**

(12) # FASCICULE DE BREVET EUROPEEN

(45) Date de publication du fascicule du brevet:
**24.05.89**

(51) Int. Cl.⁴: **C 07 H 9/02, A 23 L 1/00**

(21) Numéro de dépôt: **84903067.1**

(22) Date de dépôt: **08.08.84**

(86) Numéro de dépôt international:
**PCT/FR 84/00186**

(87) Numéro de publication internationale:
**WO 85/00814 (28.02.85 Gazette 85/5)**

(54) **PROCEDE DE CYCLO-DESHYDRATATION DES CETOSES, ANHYDRIDES OBTENUS ET LEUR UTILISATION COMME ADDITIFS ALIMENTAIRES.**

(30) Priorité: **08.08.83 FR 8313031**
**08.08.83 FR 8313032**

(43) Date de publication de la demande:
**28.08.85 Bulletin 85/35**

(45) Mention de la délivrance du brevet:
**24.05.89 Bulletin 89/21**

(84) Etats contractants désignés:
**AT BE CH DE FR GB LI LU NL SE**

(73) Titulaire: **BEGHIN-SAY SOCIETE ANONYME,**
**F-59239 Thumeries (FR)**

(72) Inventeur: **DEFAYE, Jacques, 202, chemin du Vercors,**
**F-38330 Saint Ismier (FR)**
Inventeur: **GADELLE, Andrée, 23, Hameau Fleuri,**
**Montbonot F-38330 Saint Ismier (FR)**
Inventeur: **PEDERSEN, Christian, Bredesvinget 18,**
**DK-2830 Virum (DK)**

(74) Mandataire: **Quéré, Jean Pierre, BEGHIN-SAY Service**
**Propriété Industrielle 54, Avenue Hoche, F-75008 Paris**
**(FR)**

(56) Documents cités:
**Chemical Abstracts, Vol. 90, No. 21, 21 May 1979,**
**Columbus, Ohio (US), A. Soler et al.: "Acid treatment of**
**ketohexoses", see page 627, abstract 16884, An. Univ.**
**Murcia Cien. 1975, (published in 1978) (1-2-3-4)**
**Chemical Abstracts, Vol. 85, No. 6, 9 August 1976,**
**Columbus, Ohio (US), V.S. Velasco et al.:**
**"Carbohydrate changes in invert sirups", see page 110,**
**abstract 34941z, U.S. Agric. Res. Serv. South. Reg.**
**(Rep) ARS-S-88, 138-54**
**J. DEFAYE et coll., Carbohydrate Research, 136 81985)**
**53-65**

## Description

L'invention a pour objet un procédé de cyclo-déshydratation des cétoses, les produits obtenus et leur utilisation comme additifs alimentaires.

Elle se rapporte plus particulièrement au domaine des produits de remplacement des sucres alimentaires.

Des produits de remplacement des sucres alimentaires sont recherchés depuis longtemps afin d'éviter les conséquences fâcheuses pour l'organisme d'une surconsommation de ces derniers.

Ainsi il a déjà été proposé d'utiliser des produits qui, tout en ayant un pouvoir sucrant plus ou moins marqué, ont la particularité de n'être pratiquement pas métabolisables par l'organisme humain. Ces produits sont également appelés «sucres alimentaires non caloriques». Parmi ceux-ci on connaît depuis longtemps l'important pouvoir sucrant d'un dipeptide: l'ester méthylique de l'α-aspartyl phénylalanine (ou aspartame) décrit notamment dans le brevet français N° 1577545.

Un des problèmes liés à l'utilisation comme édulcorant de l'aspartame réside dans le fait que ce composé, vu son fort pouvoir sucrant, ne peut être employé qu'en faible quantité. Par ailleurs, il ne se présente pas sous forme cristalline. Il est donc nécessaire, pour rendre le produit acceptable par le consommateur, de l'associer à un support neutre.

Le but de l'invention est donc de proposer des analogues des glucides dont certains sont des produits nouveaux en soi et qui, du fait de leur caractéristique non assimilable par l'organisme, peuvent entrer, soit seuls, soit en mélange, dans la composition d'un sucre alimentaire non calorique ou qui peuvent être utilisés comme support pour un édulcorant de synthèse ou classique de manière à pouvoir former une association ayant un pouvoir sucrant égal ou supérieur mais toujours non calorique.

Un autre but de l'invention est de proposer un procédé de synthèse avantageux de ces produits.

Le procédé de cyclo-déshydratation des glucides, dont la molécule comporte au moins un motif de type cétose, est caractérisé en ce que l'on fait réagir le glucide, sans addition d'eau, avec le fluorure d'hydrogène utilisé comme solvant du glucide, et en ce qu'on élimine le fluorure d'hydrogène par voie sèche.

On emploie le fluorure d'hydrogène, car cet halogénure permet d'obtenir des produits avec des rendements excellents. Il conduit à des rendements pratiquement quantitatifs contrairement aux procédés de l'art antérieur, voir par exemple l'article mentionné dans Chemical Abstracts Vol. 90, N° 21, 21 May 1979, A. Soler et al «Acid treatment of ketohexoses» ou bien l'article mentionné dans Chemical Abstracts, Vol. 85, N° 6, 9 August 1976, V.S. Velasco et al «Carbohydrate changes in invert sirups», où la réaction du D-fructose, de l'inuline et du L-sorbose en présence d'acides minéraux aqueux, dont en particulier l'acide chlorydrique, conduit à des mélanges de dianhydrides d'hexuloses, avec des rendements qui ne dépassent pas 50%.

Les glucides dont la molécule comporte au moins un motif de type cétose peuvent être des cétotétroses, des cétopentoses ou des cétohexoses. Ils peuvent être sous forme monomère ou polymère, ce dernier terme incluant les dimères.

Les glucides qui comportent un motif cétopentose peuvent être avantageusement choisis parmi le xylulose ou un polymère de celui-ci.

Les glucides qui comportent un motif cétohexose sont les glucides préférés pour mettre en œuvre le procédé selon l'invention. Parmi ceux-ci on peut citer le fructose, l'inuline, le sorbose.

L'invention comprend également les glucides précédemment cités dont la molécule comporte un ou plusieurs substituants de type éther, ester, acétal ou autres dans une position telle qu'ils ne gênent pas la réaction de cyclo-déshydratation.

Parmi tous ces composés on préfèrera les glucides dans lesquels l'un au moins des cycles saccharidiques est le fructose ou le sorbose.

La réaction est effectuée sans addition d'eau. Cela ne signifie pas qu'elle se déroule sous condition anhydre car comme il s'agit d'un procédé de déshydratation, de l'eau est automatiquement formée à mesure que la réaction évolue vers les composés déshydratés. En outre, les produits de départ peuvent être partiellement hydratés.

Toutefois, la proportion d'eau en fin de réaction ne doit pas excéder 10% en poids du mélange réactionnel final afin de ne pas diminuer le rendement en composés déshydratés.

Selon une variante préférée, il est néanmoins préférable que le fluorure d'hydrogène soit introduit sec. De même le sucre à réagir sera préalablement séché de sorte qu'il ne contienne plus d'eau mis à part l'eau de cristallisation.

On peut mettre en contact le glucide avec le fluorure d'hydrogène de diverses façons. Ainsi le fluorure d'hydrogène peut être mis en circulation à l'état gazeux, sous pression ou entraîné dilué dans un gaz porteur inerte tel que le dioxyde de soufre ou l'azote.

Dans la mise en œuvre de l'invention, l'efficacité du procédé est apparu directement liée à la dissolution du glucide traité dans le fluorure d'hydrogène. Il est donc préférable de dissoudre le glucide dans l'halogénure à l'état liquide et avantageusement d'assurer la dissolution complète du glucide dans l'halogénure. La température de la réaction sera avantageusement comprise entre − 20 °C et + 25 °C. On élimine ensuite l'halogénure par tout moyen approprié, notamment par évaporation.

La réaction peut être partielle et conduire néanmoins à des produits dont l'utilisation sera expliquée ci-après.

De préférence, le fluorure d'hydrogène est mis à réagir en excès en mole par rapport au nombre d'équivalents monosaccharidiques cétosiques.

Avantageusement le rapport molaire fluorure d'hydrogène: équivalent monosaccharidique cétosique est compris entre 5 et 100 et mieux entre 6 et 50.

Le procédé selon l'invention conduit à des dianhydrides saccharidiques qui peuvent être aisément isolés par cristallisation et ils sont ainsi obtenus à l'état pur avec de très bons rendements. Il est à remarquer que quel que soit le glucide de départ, la réaction semble favoriser la formation de dimère du cétose dans les dianhydrides de l'invention.

La présente invention a également pour objet les produits obtenus par le procédé qui vient d'être décrit.

L'invention conduit notamment à des dianhydrides saccharidiques dont la molécule comporte deux cycles saccharidiques reliés entre eux par deux liaisons acétaliques incluses dans un cycle dioxannique.

De préférence l'un au moins des cycles saccharidiques est un cycle de type cétose, notamment celui du fructose et ce cycle est relié au cycle dioxannique par une liaison spirannique.

Comme on le sait, les cétoses sont des glucides qui, contrairement aux aldoses, comportent au moins une fonction cétone.

Cependant, on doit comprendre ici que le cycle du fructose, ou autre cétose, est éventuellement substitué. Autrement dit, dans l'expression qui précède, on entend couvrir non seulement le fructose proprement dit, mais aussi bien les cétoses homologues, les isomères de configuration comme le sorbose, et les cétoses dont la molécule comporte un ou plusieurs substituants, de type éther, ester, acétal ou autres.

De préférence, les anhydrides sont formés en partie de dianhydrides du D-fructose.

De préférence, les anhydrides sont essentiellement formés d'un mélange de dianhydrides de di-D-fructopyranose et/ou de fructofuranose et fructopyranose.

L'invention concerne plus particulièrement les produits suivants:
- di-alpha-D-fructopyranose 1,2' : 2,1' dianhydride
- béta-D-fructofuranose béta-D-fructopyranose 1,2' : 2,1' dianhydride
- béta-D-fructofuranose béta-D-fructopyranose 2,1' : 3,2' dianhydride
- Dianhydrides de sorbose.

L'invention a également pour objet l'utilisation des produits ci-dessus éventuellement obtenus par le procédé décrit précédemment comme additifs alimentaires.

Il est remarquable que les produits selon l'invention soit à la fois non toxiques et non hydrolysables, de sorte qu'ils constituent des aliments non assimilables. De plus, de par leur structure, il est clair que même s'il leur arrivait d'être détruits dans l'organisme, leur hydrolyse ne conduirait jamais qu'à des produits non toxiques, constitués par des molécules de sucre, notamment le fructose. La présence de ces produits dans le sucre alimentaire permet de diminuer le pouvoir calorique du sucre et des aliments auxquels ils sont incorporés.

Les produits selon l'invention peuvent être obtenus par déshydratation des sucres connus. Si par exemple il s'agit du saccharose, une déshydratation, même seulement partielle, conduit à un sucre moins calorique que le produit de départ. L'invention a donc également pour objet un procédé de traitement des sucres pour diminuer leur pouvoir calorique, caractérisé en ce qu'il consiste essentiellement à assurer une déshydratation au moins partielle du sucre dans des conditions induisant la formation de liaisons acétaliques entre les cycles saccharidiques.

Par ailleurs, ces produits présentent pour certains un goût sucré ou pour d'autres ne présentent aucun goût (goût neutre). Ils se présentent sous forme cristallisée et sont solubles dans l'eau. Ils peuvent être donc avantageusement associés à un édulcorant bien connu comme l'aspartame – l'acesulfam – la saccharine ou similaire.

L'invention a donc également pour objet un produit alimentaire comportant en poids 1% à 10% d'aspartame ou similaire et 90% à 99% d'un des composés de l'invention.

L'invention est illustrée par les exemples particuliers de mise en œuvre ci-dessous:

EXEMPLE I

De l'inuline provenant de tubercules de dahlia Sigma (5 g), refroidie à 0 °C, est additionnée de fluorure d'hydrogène (10 ml), également refroidi. La solution est laissée pendant 45 minutes environ à température ambiante (20 °C), puis le fluorure d'hydrogène est évaporé par dépression ou entraînement par un courant gazeux. Le résidu est repris par l'éther.

On récupère 4,8 g d'un produit constitué de dianhydrides du D-fructose qui sont identifiés par chromatographie en phase gazeuse, sous forme des produits méthylés. Le produit apparaît formé d'un mélange comprenant, en pourcentage pondéral du poids total:

- 12% de (1): di-alpha-D-fructopyranose 1,2' : 2,1' dianhydride
  Point de fusion: 239–295 °C
  Pouvoir rotatoire $[\alpha]_D$ : −46°
- 14% de (2): di-béta-D-fructopyranose 1,2' : 2,1' dianhydride
  Point de fusion: 279 °C
  Pouvoir rotatoire $[\alpha]_D$ : −300°
- 45% de (3): alpha-D-fructofuranose alpha-D-fructopyranose 1,2' : 2,1' dianhydride
  Point de fusion: 258 °C
  Pouvoir rotatoire $[\alpha]_D$ : −39°
- 4% de (4): béta-D-fructofuranose béta-D-fructopyranose 1,2' : 2,1' dianhydride
  Point de fusion: 240 °C
  Pouvoir rotatoire $[\alpha]_D$ : −182°
- 18% de (5): béta-D-fructofuranose béta-D-fructopyranose 2,1' : 3,2' dianhydride
  Point de fusion: 206 °C
  Pouvoir rotatoire $[\alpha]_D$ : −58,5°.

EXEMPLE II

Du D-fructose (20 g) est traité par du fluorure d'hydrogène HF (20 ml) à − 10 °C pendant trois minutes, puis le fluorure d'hydrogène est éliminé par évaporation à l'air par dépression. Le résidu est additionné d'éther diéthylique refroidi. Le précipité est décanté et repris plusieurs fois à l'éther diéthylique, puis décanté et filtré.

On récupère 18,4 g d'un produit constitué d'un mélange de dianhydrides du $\underline{D}$-fructose qui sont identifiés par chromatographie en phase gazeuse et désignés comme dans l'Exemple I. Le mélange comprend, en pourcentage de poids par rapport à son poids total:

- 14% de (1)
- 20% de (2)
- 58% de (3)
- 7% de (5)

En faisant varier les conditions, soit en particulier la quantité de HF (1 ml pour 1 g au minimum jusqu'à 10 ml pour 1 g), la température (− 10 °C jusqu'à 20 °C) et le temps (de 2 minutes à 5 h), on obtient des sucres essentiellement constitués des cinq mêmes composés déjà identifiés dans l'Exemple I, avec toutefois des proportions diverses, notamment dans les distributions comprises dans les limites ci-dessous:

- de 10% à 20% de (1)
- de 10% à 20% de (2)
- de 30% à 60% de (3)
- de 4% à 8% de (4)
- de 10% à 30% de (5)

EXEMPLE III

Le mélange (18,4 g) obtenu dans l'exemple II (1er §) est repris par du méthanol (20 ml) et mis à cristalliser. On récupère les composés (1) et (2) directement (4 g).

Les eaux-mères amenées à sec sont uniquement composées des dérivés (3), (4), (5). Le dérivé (3), dérivé majoritaire du mélange (40%), est cristallisé par addition de cristaux formant germes aux eaux-mères reprises par de l'éthanol. La pureté du produit est de l'ordre de 98%.

En opérant de la même manière, mais sur un mélange obtenu selon l'Exemple II, dans des conditions différentes, on obtient ce composé (3) avec un rendement s'élevant à 60%.

EXEMPLE IV

En opérant à froid comme dans l'Exemple I, on traite du L-sorbose (10 g) par HF (10 ml) pendant 1 minute à − 10 °C. Le produit est précipité à l'éther diéthylique. On en récupère 9,2 g. Il s'agit d'un mélange de plusieurs composés, qui sont identifiés comme étant tous des dianhydrides du L-sorbose. On identifie ainsi successivement, par chromatographie en phase gazeuse et spectrométrie de résonance magnétique du carbone, les composés suivants:

- di-alpha-$\underline{L}$-sorbopyranose 1,2' : 2,1' dianhydride
- di-béta-$\underline{L}$-sorbopyranose 1,2' : 2,1' dianhydride
- alpha-$\underline{L}$-sorbofuranose alpha-$\underline{L}$-sorbopyranose 1,2' : 2,1' dianhydride
- alpha-$\underline{L}$-sorbofuranose béta-$\underline{L}$-sorbopyranose 1,2' : 2,1' dianhydride
- alpha-$\underline{L}$-sorbofuranose béta-$\underline{L}$-sorbopyranose 1,2' : 3,1' dianhydride

Les produits peuvent être isolés de la même façon que dans le cas du $\underline{D}$-fructose (Exemple II), par addition de méthanol, puis d'éthanol.

EXEMPLE V

Du saccharose (5 g) est additionné de fluorure d'hydrogène (10 ml) à 0 °C. On laisse réagir le mélange pendant 2,5 minutes, puis le produit est précipité à l'éther. On récupère 4,8 g.

Le mélange ainsi obtenu contient du fluorure de glucosyle (analyse par résonance magnétique nucléaire au $^{13}$C). Une étude des produits méthylés permet de noter la présence de dianhydrides du $\underline{D}$-fructose; les cinq composés définis dans l'Exemple I sont présents.

Du point de vue quantitatif, le mélange contient 18% de $\underline{D}$-fructose, sous forme de ces dianhydrides.

Par un tel traitement par le fluorure d'hydrogène, le saccharose de départ est donc rendu partiellement non hydrolysable enzymatiquement et son pouvoir calorique est diminué.

EXEMPLE VI

Les produits des exemples I à IV sont examinés dans les propriétés permettant d'en faire des substituts du saccharose dans l'alimentation.

Ces produits sont non toxiques. Ils sont tous solubles dans l'eau et les sirops obtenus présentent une viscosité variable avec la concentration, comme le sucre courant.

A titre d'exemple, le dernier mélange d'anhydrides du $\underline{D}$-fructose obtenu selon l'Exemple II n'est pas hydrolysé par HCl 0,01 N à 37 °C pendant 0,5 heure. De façon générale, à 20 °C en présence d'acide sulfurique 1 N, la vitesse d'hydrolyse des différents composés est environ 1000 fois inférieure à celle du saccharose.

Si par ailleurs, on soumet un mélange partiellement traité contenant des dianhydrides à une fermentation par la levure de bière, les sucres non transformés sont sensibles à la levure et peuvent être éliminés, alors que les dianhydrides de l'invention ne sont pas attaqués.

EXEMPLE VIII

On traite 5 g de sirop de xylulose par 10 ml de fluorure d'hydrogène liquide pendant 2 minutes à − 10 °C. La solution est ensuite additionnée d'éther éthylique. Après décantation on recueille 4,5 g des produits ci-après:

- di-alpha-$\underline{D}$-xylulofuranose 1,2' − 2,1' dianhydride
- di-béta-$\underline{D}$-xylulofuranose 1,2' − 2,1' dianhydride
- alpha-$\underline{D}$-xylulofuranose béta-D-xylulofuranose 1,2' − 2,1' dianhydride
- alpha-$\underline{D}$-xylulofuranose béta-D-xylulofuranose 1,2' − 3,1' dianhydride

**Revendications pour les Etats contractants BE, CH, DE, FR, GB, LI, LU, NL, SE**

1. Procédé de cyclo-déshydratation des glucides, dont la molécule comporte au moins un motif de type cétose, caractérisé en ce que l'on fait réagir le glucide, sans addition d'eau, avec le fluorure d'hydrogène utilisé comme solvant du glucide, puis on élimine le fluorure d'hydrogène par voie sèche.

2. Procédé selon la revendication 1, caractérisé en ce que le cétose est un fructose.

3. Procédé selon la revendication 2, caractérisé en ce que le glucide de départ est choisi parmi le fructose, le saccharose, l'inuline.

4. Procédé selon la revendication 1, caractérisé en ce que le cétose est un sorbose.

5. Procédé selon la revendication 1, caractérisé en ce que le rapport molaire fluorure d'hydrogène : équivalent monosaccaridique cétosique est compris entre 5 et 100 de préférence entre 6 et 50.

6. Procédé selon la revendication 1, caractérisé en ce que l'on opère en conditions telles que la quantité d'eau présente dans le milieu réactionnel avant élimination du fluorure d'hydrogène soit inférieure à 10% en poids.

7. Procédé selon la revendication 1, caractérisé en ce que la réaction est effectuée avec le fluorure d'hydrogène à l'état liquide.

8. Procédé selon la revendication 7, caractérisé en ce que la température du milieu réactionnel est comprise entre − 20 °C et + 25 °C.

9. Procédé selon la revendication 7, caractérisé en ce qu'on élimine le fluorure d'hydrogène, après la réaction, par évaporation.

10. Béta-$\underline{D}$-fructofuranose béta-$\underline{D}$-fructopyranose 2,1′ : $\overline{3}$,2′ dianhydride de point de fusion 206 °C et de pouvoir rotatoire $[\alpha]_D$ : − 58,5°.

11. Mélange de dianhydrides de glucides contenant au moins un motif du type cétose, caractérisé en ce qu'il contient au moins le béta-$\underline{D}$-fructofuranose béta-$\underline{D}$-fructopyranose 2,1′ : 3,2′ dianhydride selon la revendication 10.

12. Mélange de dianhydrides selon la revendication 11, caractérisé en ce qu'il est obtenu à partir de la cyclodéshydratation d'au moins un glucide tel que l'inuline ou le $\underline{D}$-fructose, réalisée à l'aide du fluorure d'hydrogène.

13. Alpha-$\underline{L}$-sorbofuranose alpha-$\underline{L}$-sorbopyranose 1,2′ : 2,$\overline{1}$′ dianhydride.

14. Mélange de dianhydrides de glucides contenant au moins un motif cétose caractérisé en ce qu'il contient au moins le alpha-$\underline{L}$-sorbofuranose alpha-$\underline{L}$-sorbopyranose 1,2′ : 2,1′ dianhydride.

15. Mélange de dianhydrides, selon la revendication 14, caractérisé en ce qu'il est obtenu à partir de la cyclodéshydratation d'au moins un glucide tel que le $\underline{L}$-sorbose, réalisée à l'aide du fluorure d'hydrogène.

16. Application des produits selon l'une quelconque des revendications 10 à 15 comme produits ou additifs alimentaires non caloriques.

**Jeu de revendications pour l'état contractant AT**

1. Procédé de cyclo-déshydratation des glucides, dont la molécule comporte au moins un motif de type cétose, caractérisé en ce que l'on fait réagir le glucide, sans addition d'eau, avec le fluorure d'hydrogène utilisé comme solvant du glucide, puis on élimine le fluorure d'hydrogène par voie sèche.

2. Procédé selon la revendication 1, caractérisé en ce que le cétose est un fructose.

3. Procédé selon la revendication 2, caractérisé en ce que le glucide de départ est choisi parmi le fructose, le saccharose, l'inuline.

4. Procédé selon la revendication 1, caractérisé en ce que le cétose est un sorbose.

5. Procédé selon la revendication 1, caractérisé en ce que le rapport molaire fluorure d'hydrogène : équivalent monosaccharidique cétosique est compris entre 5 et 100 de préférence entre 6 et 50.

6. Procédé selon la revendication 1, caractérisé en ce que l'on opère en conditions telles que la quantité d'eau présente dans le milieu réactionnel avant élimination du fluorure d'hydrogène soit inférieure à 10% en poids.

7. Procédé selon la revendication 1, caractérisé en ce que la réaction est effectuée avec le fluorure d'hydrogène à l'état liquide.

8. Procédé selon la revendication 7, caractérisé en ce que la température du milieu réactionnel est comprise entre − 20 °C et + 25 °C.

9. Procédé selon la revendication 7, caractérisé en ce qu'on élimine le fluorure d'hydrogène, après la réaction, par évaporation.

10. Procédé de fabrication de produits ou additifs alimentaires non caloriques selon l'une des revendications 1 à 9.

**Claims for the contracting states BE, CH, DE, FR, GB, LI, LU, NL, SE**

1. Process for the cyclodehydration of glucides, whose molecules contain at least one ketose-type unit, characterized in that the glucide is reacted, without addition of water, with the hydrogen fluoride used as solvent for the glucide, then the hydrogen fluoride is eliminated by dry means.

2. Process according to Claim 1, characterized in that the ketose is a fructose.

3. Process according to Claim 2, characterized in that the initial glucide is selected among fructose, sucrose, inulin.

4. Process according to Claim 1, characterized in that the ketose is a sorbose.

5. Process according to Claim 1, characterized in that the molar ratio of hydrogen fluoride to monosaccharidic ketosic equivalent is between 5 and 100, preferably between 6 and 50.

6. Process according to Claim 1, characterized in that the operating conditions are such that the quantity of water present in the reaction mixture before elimination of the hydrogen fluoride is less than 10% by weight.

7. Process according to Claim 1, characterized in that the reaction is carried out with hydrogen fluoride in the liquid state.

8. Process according to Claim 7, characterized in that the temperature of the reaction mixture is between −20 °C and +25 °C.

9. Process according to Claim 7, characterized in that the hydrogen fluoride is eliminated, after the reaction, by evaporation.

10. Beta-$\underline{D}$-fructofuranose beta-$\underline{D}$-fructopyranose 2,1′; 3,$\overline{2}$′-dianhydride of melting point 206 °C and of optical rotation $[\alpha]_D$ : −58.5°.

11. Mixture of dianhydrides of glucides containing at least one ketose-type unit, characterized in that it at least contains beta-D-fructofuranose beta-D-fructopyranose 2,1'; 3,2'-dianhydride according to Claim 10.

12. Mixture of dianhydrides according to Claim 11, characterized in that it is obtained by the cyclodehydration of at least one glucide such as inulin or D-fructose, effected by means of hydrogen fluoride.

13. Alpha-L-sorbofuranose alpha-L-sorbopyranose 1,2'; 2,1'-dianhydride.

14. Mixture of dianhydrides of glucides containing at least one ketose unit, characterized in that it at least contains alpha-L-sorbofuranose alpha-L-sorbopyranose 1,2'; 2,1'-dianhydride.

15. Mixture of dianhydrides, according to Claim 14, characterized in that it is obtained from the cyclodehydration of at least one glucide such as L-sorbose, effected by means of hydrogen fluoride.

16. Application of the products according to any one of Claims 10 to 15 as non-caloric food products or additives.

**Claims for the contracting state AT**

1. Process for the cyclodehydration of glucides, whose molecules contain at least one ketose-type unit, characterized in that the glucide is reacted, without addition of water, with the hydrogen fluoride used as solvent for the glucide, then the hydrogen fluoride is eliminated by dry means.

2. Process according to Claim 1, characterized in that the ketose is a fructose.

3. Process according to Claim 2, characterized in that the initial glucide is selected among fructose, sucrose, inulin.

4. Process according to Claim 1, characterized in that the ketose is a sorbose.

5. Process according to Claim 1, characterized in that the molar ratio of hydrogen fluoride to monosaccharidic ketosic equivalent is between 5 and 100, preferably between 6 and 50.

6. Process according to Claim 1, characterized in that the operating conditions are such that the quantity of water present in the reaction mixture before elimination of the hydrogen fluoride is less than 10% by weight.

7. Process according to Claim 1, characterized in that the reaction is carried out with hydrogen fluoride in the liquid state.

8. Process according to Claim 7, characterized in that the temperature of the reaction mixture is between −20°C and +25°C.

9. Process according to Claim 7, characterized in that the hydrogen fluoride is eliminated, after the reaction, by evaporation.

10. Process for the manufacture of non-caloric food products or additives according to one of Claims 1 to 9.

**Ansprüche für die Vertragsstaaten BE, CH, DE, FR, GB, LI, LU, NL, SE**

1. Verfahren zur Cyclo-Entwässerung von Kohlenhydraten, deren Molekül mindestens ein Motiv

vom Ketosetyp enthält, dadurch gekennzeichnet, daß man das Kohlenhydrat ohne Hinzufügung von Wasser mit Fluorwasserstoff, der als Lösungsmittel für das Kohlenhydrat verwendet wird, reagieren läßt, und dann den Fluorwasserstoff durch Trocknung entfernt.

2. Verfahren nach Anspruch 1, dadurch gekennzeichnet, daß die Ketose eine Fructose ist.

3. Verfahren nach Anspruch 2, dadurch gekennzeichnet, daß das Ausgangskohlenhydrat Fructose, Saccharose oder Inulin ist.

4. Verfahren nach Anspruch 1, dadurch gekennzeichnet, daß die Ketose eine Sorbose ist.

5. Verfahren nach Anspruch 1, dadurch gekennzeichnet, daß das molare Verhältnis Fluorwasserstoff : Monosaccharidäquivalente vom Ketosetyp sich zwischen 5 und 100, vorzugsweise zwischen 6 und 50, bewegt.

6. Verfahren nach Anspruch 1, dadurch gekennzeichnet, daß man unter solchen Bedingungen arbeitet, daß die in dem Reaktionsmilieu anwesende Wassermenge vor Entfernung des Fluorwasserstoffes unterhalb von 10 Gew.-% liegt.

7. Verfahren nach Anspruch 1, dadurch gekennzeichnet, daß die Reaktion mit flüssigem Fluorwasserstoff durchgeführt wird.

8. Verfahren nach Anspruch 7, dadurch gekennzeichnet, daß die Temperatur des Reaktionsmilieus zwischen −20 und +25°C liegt.

9. Verfahren nach Anspruch 7, dadurch gekennzeichnet, daß man den Fluorwasserstoff nach der Reaktion durch Verdampfen entfernt.

10. Beta-D-Fructofuranose beta-D-Fructopyranose 2,1' : 3,2' Dianhydrid, mit einem Schmelzpunkt von 206°C und einem Drehvermögen $[\alpha]_D$ : −58,5°.

11. Gemisch von Kohlenhydratdianhydriden mit mindestens einem Motiv vom Ketosetyp, dadurch gekennzeichnet, daß es mindestens das beta-D-Fructofuranose beta-D-Fructopyranose 2,1' : 3,2' Dianhydrid nach Anspruch 10 enthält.

12. Dianhydridgemisch nach Anspruch 11, dadurch gekennzeichnet, daß es teilweise durch Cycloentwässerung von mindestens einem Kohlenhydrat wie dem Inulin oder der D-Fructose mit Hilfe von Fluorwasserstoff erhalten wird.

13. Alpha-L-Sorbofuranose alpha-L-Sorbopyranose 1,2' : 2,1' Dianhydrid.

14. Gemisch von Kohlenhydratdianhydriden mit mindestens einem Motiv vom Typ Ketose, dadurch gekennzeichnet, daß es mindestens alpha-L-Sorbofuranose alpha-L-Sorbopyranose 1,2' : 2,1' Dianhydrid enthält.

15. Dianhydridgemisch nach Anspruch 14, dadurch gekennzeichnet, daß es durch Cycloentwässerung von mindestens einem Kohlenhydrat wie der L-Sorbose unter Zuhilfenahme von Fluorwasserstoff erhalten wird.

16. Verwendung der Produkte nach einem der Ansprüche 10 bis 15 als Nahrungsprodukte oder -zusätze, die keine Kalorien enthalten.

**Ansprüche für den Vertragsstaat AT**

1. Verfahren zur Cyclo-Entwässerung von Kohlenhydraten, deren Molekül mindestens ein Motiv

vom Ketosetyp enthält, dadurch gekennzeichnet, daß man das Kohlenhydrat ohne Hinzufügung von Wasser mit Fluorwasserstoff, der als Lösungsmittel für das Kohlenhydrat verwendet wird, reagieren läßt, und dann den Fluorwasserstoff durch Trocknung entfernt.

2. Verfahren nach Anspruch 1, dadurch gekennzeichnet, daß die Ketose eine Fructose ist.

3. Verfahren nach Anspruch 2, dadurch gekennzeichnet, daß das Ausgangskohlenhydrat Fructose, Saccharose oder Inulin ist.

4. Verfahren nach Anspruch 1, dadurch gekennzeichnet, daß die Ketose eine Sorbose ist.

5. Verfahren nach Anspruch 1, dadurch gekennzeichnet, daß das molare Verhältnis Fluorwasserstoff : Monosaccharidäquivalente vom Ketosetyp sich zwischen 5 und 100, vorzugsweise zwischen 6 und 50, bewegt.

6. Verfahren nach Anspruch 1, dadurch gekennzeichnet, daß man unter solchen Bedingungen arbeitet, daß die in dem Reaktionsmilieu anwesende Wassermenge vor Entfernung des Fluorwasserstoffes unterhalb von 10 Gew.-% liegt.

7. Verfahren nach Anspruch 1, dadurch gekennzeichnet, daß die Reaktion mit flüssigem Fluorwasserstoff durchgeführt wird.

8. Verfahren nach Anspruch 7, dadurch gekennzeichnet, daß die Temperatur des Reaktionsmilieus zwischen $-20$ und $+25\,°C$ liegt.

9. Verfahren nach Anspruch 7, dadurch gekennzeichnet, daß man den Fluorwasserstoff nach der Reaktion durch Verdampfen entfernt.

10. Verfahren zur Herstellung von nicht kalorienhaltigen Nahrungsmittelprodukten oder -zusätzen nach einem der Ansprüche 1–9.